(19)

Europäisches Patentamt

European Patent Office

Office européen des brevets

(11) **EP 2 550 975 A1**

(12) **EUROPEAN PATENT APPLICATION**

(43) Date of publication:
**30.01.2013 Bulletin 2013/05**

(21) Application number: **11305999.2**

(22) Date of filing: **29.07.2011**

(51) Int Cl.:
*A61K 47/48* (2006.01)      *A61K 39/395* (2006.01)
*C07K 16/28* (2006.01)      *A61P 35/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANOFI**
**75008 Paris (FR)**

(72) Inventors:
• **Besret, Laurent**
  **75008 Paris (FR)**

• **Carrez, Chantal**
  **75008 Paris (FR)**
• **Payrard, Sandrine**
  **75008 Paris (FR)**

(74) Representative: **Bouvet, Philippe et al**
**Sanofi Aventis**
**Patents France**
**174 avenue de France**
**75013 Paris (FR)**

(54) **Combination therapy for the treatment of CD19+ B-cell malignancies symptoms comprising an anti-CD19 maytansinoid immunoconjugate and rituximab**

(57)      A combination of an anti-CD19 maytansinoid immunoconjugate and rituximab is used for treating CD19+ CD20+ B-cell malignancies symptom, in particular Non-Hodgkin's lymphoma.

**EP 2 550 975 A1**

SEQUENCE LISTING

<110> SANOFI

<120> COMBINATION THERAPY FOR THE TREATMENT OF CD19+ B-CELL
MALIGNANCIES SYMPTOMS COMPRISING AN ANTI-CD19 MAYTANSINOID
IMMUNOCONJUGATE (SAR3419) AND RITUXIMAB

<130> FR2011/053

<160> 8

<170> PatentIn version 3.3

<210> 1
<211> 10
<212> PRT
<213> Mus musculus

<220>
<221> misc_feature
<223> CDR L1

<400> 1

Ser Ala Ser Ser Gly Val Asn Tyr Met His
1               5                   10

<210> 2
<211> 7
<212> PRT
<213> Mus musculus

<220>
<221> misc_feature
<223> CDR L2 sequence

<400> 2

Asp Thr Ser Lys Leu Ala Ser
1               5

<210> 3
<211> 7
<212> PRT
<213> Mus musculus

<220>
<221> misc_feature
<223> CDR L3 sequence

<400> 3

His Gln Arg Gly Ser Tyr Thr
1               5

<210> 4
<211> 5
<212> PRT

```
<213>   Mus musculus


<220>
<221>   misc_feature
<223>   CDR H1 sequence

<400>   4

Ser Asn Trp Met His
1               5


<210>   5
<211>   10
<212>   PRT
<213>   Mus musculus


<220>
<221>   misc_feature
<223>   CDR H2 sequence

<400>   5

Glu Ile Asp Pro Ser Asp Ser Tyr Thr Asn
1               5                   10


<210>   6
<211>   11
<212>   PRT
<213>   Mus musculus


<220>
<221>   misc_feature
<223>   CDR H3 sequence

<400>   6

Gly Ser Asn Pro Tyr Tyr Tyr Ala Met Asp Tyr
1               5                   10


<210>   7
<211>   211
<212>   PRT
<213>   Artificial

<220>
<223>   huB4 Light Chain Sequence

<400>   7

Glu Ile Val Leu Thr Gln Ser Pro Ala Ile Met Ser Ala Ser Pro Gly
1               5                   10                  15


Glu Arg Val Thr Met Thr Cys Ser Ala Ser Ser Gly Val Asn Tyr Met
            20                  25                  30


His Trp Tyr Gln Gln Lys Pro Gly Thr Ser Pro Arg Arg Trp Ile Tyr
            35                  40                  45
```

```
Asp Thr Ser Lys Leu Ala Ser Gly Val Pro Ala Arg Phe Ser Gly Ser
    50                  55                  60

Gly Ser Gly Thr Asp Tyr Ser Leu Thr Ile Ser Ser Met Glu Pro Glu
65                  70                  75                  80

Asp Ala Ala Thr Tyr Tyr Cys His Gln Arg Gly Ser Tyr Thr Phe Gly
                85                  90                  95

Gly Gly Thr Lys Leu Glu Ile Lys Arg Thr Val Ala Ala Pro Ser Val
            100                 105                 110

Phe Ile Phe Pro Pro Ser Asp Glu Gln Leu Lys Ser Gly Thr Ala Ser
        115                 120                 125

Val Val Cys Leu Leu Asn Asn Phe Tyr Pro Arg Glu Ala Lys Val Gln
    130                 135                 140

Trp Lys Val Asp Asn Ala Leu Gln Ser Gly Asn Ser Gln Glu Ser Val
145                 150                 155                 160

Thr Glu Gln Asp Ser Lys Asp Ser Thr Tyr Ser Leu Ser Ser Thr Leu
                165                 170                 175

Thr Leu Ser Lys Ala Asp Tyr Glu Lys His Lys Val Tyr Ala Cys Glu
            180                 185                 190

Val Thr His Gln Gly Leu Ser Ser Pro Val Thr Lys Ser Phe Asn Arg
        195                 200                 205

Gly Glu Cys
    210

<210>  8
<211>  450
<212>  PRT
<213>  Artificial

<220>
<223>  huB4 Heavy Chain Sequence

<400>  8

Gln Val Gln Leu Val Gln Pro Gly Ala Glu Val Val Lys Pro Gly Ala
1                   5                   10                  15

Ser Val Lys Leu Ser Cys Lys Thr Ser Gly Tyr Thr Phe Thr Ser Asn
            20                  25                  30

Trp Met His Trp Val Lys Gln Ala Pro Gly Gln Gly Leu Glu Trp Ile
```

35　　　　　　　40　　　　　　　45

Gly Glu Ile Asp Pro Ser Asp Ser Tyr Thr Asn Tyr Asn Gln Asn Phe
50　　　　　　　55　　　　　　　60

Gln Gly Lys Ala Lys Leu Thr Val Asp Lys Ser Thr Ser Thr Ala Tyr
65　　　　　　　70　　　　　　　75　　　　　　　80

Met Glu Val Ser Ser Leu Arg Ser Asp Asp Thr Ala Val Tyr Tyr Cys
85　　　　　　　90　　　　　　　95

Ala Arg Gly Ser Asn Pro Tyr Tyr Tyr Ala Met Asp Tyr Trp Gly Gln
100　　　　　　　105　　　　　　　110

Gly Thr Ser Val Thr Val Ser Ser Ala Ser Thr Lys Gly Pro Ser Val
115　　　　　　　120　　　　　　　125

Phe Pro Leu Ala Pro Ser Ser Lys Ser Thr Ser Gly Gly Thr Ala Ala
130　　　　　　　135　　　　　　　140

Leu Gly Cys Leu Val Lys Asp Tyr Phe Pro Glu Pro Val Thr Val Ser
145　　　　　　　150　　　　　　　155　　　　　　　160

Trp Asn Ser Gly Ala Leu Thr Ser Gly Val His Thr Phe Pro Ala Val
165　　　　　　　170　　　　　　　175

Leu Gln Ser Ser Gly Leu Tyr Ser Leu Ser Ser Val Val Thr Val Pro
180　　　　　　　185　　　　　　　190

Ser Ser Ser Leu Gly Thr Gln Thr Tyr Ile Cys Asn Val Asn His Lys
195　　　　　　　200　　　　　　　205

Pro Ser Asn Thr Lys Val Asp Lys Lys Val Glu Pro Lys Ser Cys Asp
210　　　　　　　215　　　　　　　220

Lys Thr His Thr Cys Pro Pro Cys Pro Ala Pro Glu Leu Leu Gly Gly
225　　　　　　　230　　　　　　　235　　　　　　　240

Pro Ser Val Phe Leu Phe Pro Pro Lys Pro Lys Asp Thr Leu Met Ile
245　　　　　　　250　　　　　　　255

Ser Arg Thr Pro Glu Val Thr Cys Val Val Val Asp Val Ser His Glu
260　　　　　　　265　　　　　　　270

Asp Pro Glu Val Lys Phe Asn Trp Tyr Val Asp Gly Val Glu Val His
275　　　　　　　280　　　　　　　285

Asn Ala Lys Thr Lys Pro Arg Glu Glu Gln Tyr Asn Ser Thr Tyr Arg
290　　　　　　　295　　　　　　　300

```
Val Val Ser Val Leu Thr Val Leu His Gln Asp Trp Leu Asn Gly Lys
305             310             315             320

Glu Tyr Lys Cys Lys Val Ser Asn Lys Ala Leu Pro Ala Pro Ile Glu
            325             330             335

Lys Thr Ile Ser Lys Ala Lys Gly Gln Pro Arg Glu Pro Gln Val Tyr
            340             345             350

Thr Leu Pro Pro Ser Arg Asp Glu Leu Thr Lys Asn Gln Val Ser Leu
            355             360             365

Thr Cys Leu Val Lys Gly Phe Tyr Pro Ser Asp Ile Ala Val Glu Trp
370             375             380

Glu Ser Asn Gly Gln Pro Glu Asn Asn Tyr Lys Thr Thr Pro Pro Val
385             390             395             400

Leu Asp Ser Asp Gly Ser Phe Phe Leu Tyr Ser Lys Leu Thr Val Asp
            405             410             415

Lys Ser Arg Trp Gln Gln Gly Asn Val Phe Ser Cys Ser Val Met His
            420             425             430

Glu Ala Leu His Asn His Tyr Thr Gln Lys Ser Leu Ser Leu Ser Pro
            435             440             445

Gly Lys
    450
```

**Description**

The present invention relates to combinations of an anti-CD19 maytansinoid immunoconjugate and rituximab which are therapeutically useful in the treatment of CD19+ B-cell malignancies symptom.

## INTRODUCTION

B-cell Non-Hodgkin's lymphoma (B-NHL) is the fifth most common malignancy in the United States and continues to increase in incidence, especially in elderly patients. While patients with hematological malignancies have benefited over the past decade from therapeutic optimization using conventional drug therapy, a majority of patients still succumb to their disease and drug therapies remain highly toxic. Hence, future efforts towards developing new therapies to improve survival and quality of life of lymphoma patients must include strategies that specifically targets cancer cells and show improved safety and efficacy.

The rituximab (RITUXAN®; MABTHERA®) antibody is a genetically engineered chimeric murine/human monoclonal antibody directed against the CD20 antigen. Rituximab is the antibody called "C2B8" in US Patent No. 5,736,137. Rituximab is the standard initial therapy for patients with relapsed or refractory low-grade or follicular, CD20+, B-cell non-Hodgkin's lymphoma. There are a number of limitations to rituximab therapy, however. In the pivotal clinical trial that led to its approval for clinical use in the United States, only 48% of patients with relapsed follicular lymphoma responded (6% complete and 42% partial responses; McLaughlin et al., J Clin Oncol, 16: 2825-2833, 1998). Moreover, almost 60% of initially responding patients will not respond to subsequent therapy (Davis et al., J Clin Oncol, 18: 3135-3143, 2000).

Although the outcome of patients with aggressive lymphomas has improved considerably with the use of rituximab in combination with CHOP (R-CHOP), patients who experience relapse after salvage regimens have a poor prognosis. Patients may be cured with aggressive chemotherapy and autologous stem cell transplantation. Nevertheless, 40-50% of patients either are not eligible for stem cell transplantation - because of age, co-morbidities, or resistance to salvage chemotherapy- or relapse after stem cell transplantation. Prognosis in these patients is very poor, with less than 20% of patients being alive at 1 year.

There is thus still a need for novel restatement strategies to improve therapeutic efficacy for aggressive B-cell malignancies.

CD19 is the earliest differentiation antigen of the B lymphocyte lineage, expressed on most B-cells, but not detected on plasma cells, stem cells, or on normal myeloid lineage. Therefore, CD19 is expressed on tumor cells from all B cell-derived neoplasms (B-cell non-Hodgkin's lymphoma, acute lymphoblastic leukemia, chronic lymphocytic leukemia), except myeloma.

HuB4-DM4 is an antibody-drug conjugate composed of a humanized IgG1 monoclonal antibody, huB4 (Roguska et al., Proc. Natl. Acad. Sci. USA, 91: 969-973, 1994), which specifically targets the CD19 antigen, conjugated through a disulfide link to the maytansinoid derivative DM4, a potent tubulin inhibitor (US 2004/0235840). The structure of the HuB4-DM4 conjugate SAR3419 is disclosed on figure 1. After binding to the CD19 antigen, the HuB4-DM4 conjugate undergoes internalization and intracellular release of DM4.

## DESCRIPTION

It has now been found that a combination of rituximab and an anti-CD19 maytansinoid immunoconjugate is efficacious at treating B-cell malignancies symptom. Specifically, a combination of rituximab and an anti-CD19 maytansinoid immunoconjugate can be used for treating B-cell malignancies symptom in patients relapsed or resistant.

The present invention relates in a first aspect to a method of treatment of B-cell malignancies symptom in a patient in need thereof, said method comprising the step of administering to said patient a therapeutically effective amount of a combination of rituximab and an anti-CD19 maytansinoid immunoconjugate.

The present invention thus relates to a combination of rituximab and an anti-CD19 maytansinoid immunoconjugate for use in treating B-cell malignancies symptom in a patient in need thereof.

Preferably; the said B-cell malignancies symptom is a CD19+ CD20+ B-cell malignancies symptom.

The present invention also relates to the use of a combination of rituximab and an anti-CD19 maytansinoid immunoconjugate for the manufacture of a medicament for treating CD19+ CD20+ B-cell malignancies symptom in a patient in need thereof.

As used in accordance with this invention, the term "treatment" means treating a patient having a CD19+ CD20+ B-cell malignancy symptom by providing said patient with an effective amount of a combination of rituximab and anti-CD19 maytansinoid immunoconjugate the purpose of inhibiting progression of the CD19+ CD20+ B-cell malignancy symptom, growth of a tumor in such patient, eradication of the CD19+ CD20+ B-cell malignancy symptom, prolonging survival of the patient and/or palliation of the patient.

CD19+ CD20+ B-cell malignancies are defined as any malignancies expressing the CD19 cell surface antigen and the CD20 cell surface antigen. By "CD19", it is herein referred to a 95-kDa cell surface molecule expressed only by B lymphocytes and follicular dendritic cells of the hematopoietic system. Preferentially, a CD19 protein according to the invention has an amino acid sequence represented by Genbank accession number AAA69966. By "CD20", it is herein referred to a 16-kDa lymphocyte surface molecule that is widely expressed during B-cell ontogeny. Preferably, a CD20 protein according to the invention has an amino acid sequence represented by the Genbank accession number NP_ 690605. The expression of CD19 and/or CD20 can be assessed by immunochemistry or flow cytometry analysis, or by any other suitable means known to the person of skills in the art.

Said CD19+ CD20+ B-cell malignancies symptom can be a leukemia symptom, such as Acute lymphoblastic leukemia (ALL) symptom or a lymphoma symptom, such as a Non-Hodgkin's lymphoma symptom (NHL) symptom.

The Non-Hodgkin's lymphoma symptom can be a Diffuse Large B-cell lymphoma (DLBCL), a follicular lymphoma (FL), a Mantle cell lymphoma (MCL), a Marginal zone lymphoma (MZL), or a Small lymphocytic lymphoma (SLL).

In a particular embodiment of the invention, the said Non-Hodgkin's lymphoma symptom is a relapsed or refractory B-cell non-Hodgkin's lymphoma.

In another particular embodiment, the said Non-Hodgkin's lymphoma symptom is a B-cell non-Hodgkin's lymphoma expressing CD19.

In another particular embodiment, the said patient has already been treated for the Non-Hodgkin's lymphoma symptom. In a preferred embodiment, said patient has previously failed therapy, and in particular a chemotherapy and/or a rituximab therapy. Such chemotherapy is, in particular, CHOP chemotherapy, said CHOP consisting of a combination of cyclophosphamide (brand names cytoxan, neosar), adriamycin (doxorubicin / hydroxydoxorubicin), vincristine (oncovin), and prednisone (sometimes called deltasone or orasone).

In another particular embodiment, the said Non-Hodgkin's lymphoma symptom is a rituximab-resistant disease.

In another particular embodiment of this method the said patient has received a autologous or allogeneic stem cell transplant.

The anti-CD19 maytansinoid immunoconjugate of the invention comprises two primary components, a cell-binding agent and a cytotoxic agent.

As used herein, the term "cell binding agent" refers to an agent that specifically recognizes and binds the CD19 antigen on the cell surface.

In a particular embodiment, the cell-binding agent is an antibody which binds specifically to the CD19 antigen.

In a preferred embodiment, the said antibody comprises six complementary determining region (CDR), said CDR having the sequences represented in SEQ ID NOs 1 to 6.

In another preferred embodiment, the antibody comprises a light chain, wherein the sequence of the said light chain has at least 60%, preferably of at least 75%, more preferably at least 85%, still more preferably 95 % and even more preferably of at least 99% identity with the sequence displayed in SEQ ID NO. 7.

In yet another preferred embodiment, the antibody comprises a heavy chain, wherein the sequence of the said heavy chain has at least 60%, preferably of at least 75%, more preferably at least 85%, still more preferably 95 % and even more preferably of at least 99% identity with the sequence displayed in SEQ ID NO. 8.

In the most preferred embodiment, the antibody of the invention is the humanized antibody huB4 described in Roguska et al. (Proc. Natl. Acad. Sci. USA, 91: 969-973, 1994). The antibody huB4 according to the invention comprises a light chain and a heavy chain, said light chain having the sequence represented in SEQ ID NO. 7, and said heavy chain having the sequence represented in SEQ ID NO. 8.

The second component of the anti-CD19 maytansinoid immunoconjugate of the present invention is a cytotoxic agent. The term "cytotoxic agent" as used herein refers to a substance that reduces or blocks the function, or growth, of cells and/or causes destruction of cells. In a preferred embodiment, the cytotoxic agent is a maytansinoid such as DM1 or DM4. In another preferred embodiment, the cell binding agent of the present invention is covalently attached, directly or via a cleavable or non-cleavable linker, to the cytotoxic agent. Preferably, the said cell binding agent is conjugated to DM4 through a cleavable linker, most preferably through a SPDB linker. DM4 and a method of conjugating DM4 to the huB4 antibody through a SPDB linker are described in US Patent Application No. 2004/0235840.

In a particular embodiment of this method the anti-CD19 maytansinoid immunoconjugate comprises an antibody which binds specifically to the CD19 antigen conjugated to DM4 through SPDB wherein almost 3.5 molecules of DM4 are bound through the SPDB linker to each antibody molecule.

The combinations of the invention may be in the form of a kit of parts. The invention therefore includes a product containing rituximab and the anti-CD19 maytansinoid immunoconjugate of the invention as a combined preparation for simultaneous, separate or sequential delivery for the treatment of a CD19+ CD20+ malignancies symptom in a patient in need thereof. In one embodiment, a product contains rituximab and the said anti-CD19 maytansinoid immunoconjugate as a combined preparation for simultaneous, separate or sequential use in treating a CD19+ CD20+ malignancies symptom in a patient in need thereof. In one embodiment, the invention provides a pharmaceutical pack containing a course of a treatment against CD19+ CD20+ malignancies symptom for one individual patient, wherein the pack contains (a) at least one unit of rituximab and (b) at least one unit of the anti-CD19 maytansinoid immunoconjugate of the invention in unit dosage form.

In previous studies with the anti-CD19 maytansinoid immunoconjugate of the invention, it had been observed that the main toxicities were ocular, consisting mainly of reversible corneal toxicity.

Such toxicity can be alleviated, while maintaining the activity of the combination against CD19+ CD20+ malignancies symptom, by a specific dosage regimen.

In particular, the combination of an anti-CD19 maytansinoid immunoconjugate and rituximab of the invention is administered to a human patient diagnosed with a CD19+ CD20+ B-cell malignancies symptom, according to the following regimen:

(a) an initial dose of almost 55 mg/m$^2$ of the anti-CD19 maytansinoid immunoconjugate and of almost 375 mg/m$^2$ of rituximab, is first administered to the patient;

(b) a plurality of subsequent doses of almost 55 mg/m$^2$ of the anti-CD19 maytansinoid immunoconjugate of almost 375 mg/m$^2$ of rituximab, are then administered to the patient, each administration being separated in time from the other by one week.

In a preferred embodiment, the dosage regimen of the invention comprises the following steps:

(a) an initial dose of almost 55 mg/m$^2$ of the anti-CD19 maytansinoid immunoconjugate and of almost 375 mg/m$^2$ of rituximab, is first administered to the patient;

(b) a plurality of subsequent doses of almost 55 mg/m$^2$ of the anti-CD19 maytansinoid immunoconjugate of almost 375 mg/m$^2$ of rituximab, are then administered to the patient, each administration being separated in time from the other by one week; and

(c) a plurality of subsequent doses of almost 55 mg/m$^2$ of the anti-CD19 maytansinoid immunoconjugate of almost 375 mg/m$^2$ of rituximab, are further administered to the patient, each administration being separated in time from the other by two weeks.

In a further preferred embodiment, the dosage regimen of the invention comprises the following steps:

( a) an initial dose of almost 55 mg/m$^2$ of the anti-CD19 maytansinoid immunoconjugate and of almost 375 mg/m$^2$ of rituximab, is first administered to the patient;

( b) 3 subsequent doses of almost 55 mg/m$^2$ of the anti-CD19 maytansinoid immunoconjugate of almost 375 mg/m$^2$ of rituximab, are then administered to the patient, each administration being separated in time from the other by one week; and

( c) 4 subsequent doses of almost 55 mg/m$^2$ of the anti-CD19 maytansinoid immunoconjugate of almost 375 mg/m$^2$ of rituximab, are further administered to the patient, each administration being separated in time from the other by two weeks.

The present invention thus relates to a combination of an anti-CD19 maytansinoid immunoconjugate and rituximab for use in treating a CD19+ CD20+ B-cell malignancies symptom, wherein the said combination is administered according to a dosage regimen as described above.

The present invention thus also relates to methods of treatment of a CD19+ CD20+ B-cell malignancies symptom, wherein the combination of an anti-CD19 maytansinoid immunoconjugate and rituximab is administered according to a dosage regimen as described above.

The present invention thus relates to the use of a combination of an anti-CD19 maytansinoid immunoconjugate and rituximab for preparing a medicament for treating a CD19+ CD20+ B-cell malignancies symptom, wherein the said combination is administered according to a dosage regimen as described above.

The present invention also relates to pharmaceutical compositions containing the combinations according to the invention and a pharmaceutically acceptable carrier.

As used herein, "pharmaceutically acceptable carrier" includes any and all solvents, buffers, salt solutions, dispersion media, coatings, antibacterial and antifungal agents, isotonic and absorption delaying agents, and the like that are physiologically compatible. The type of carrier can be selected based upon the intended route of administration. In various embodiments, the carrier is suitable for intravenous, intraperitoneal, subcutaneous, intramuscular, topical, transdermal or oral administration. Examples of suitable carriers, diluents and/or excipients include one or more of water, saline, phosphate buffered saline, dextrose, glycerol, ethanol, and the like, as well as combination thereof. In many cases, it will be preferable to include isotonic agents, such as sugars, polyalcohols, or sodium chloride in the composition. In particular, relevant examples of suitable carrier include: (1) Dulbecco's phosphate buffered saline, pH ~ 7.4, containing or not containing about 1 mg/ml to 25 mg/ml human serum albumin, (2) 0.9% saline (0.9% w/v sodium chloride (NaCl)), and (3) 5% (w/v) dextrose; and may also contain an antioxidant such as tryptamine and a stabilizing agent such as Tween 20. Actual methods for preparing parenterally administrable compounds will be known or apparent to those skilled in the art and are described in more detail in for example, Remington's Pharmaceutical Science, 17th ed., Mack Publishing Company, Easton, Pa. (1985), and the 18th and 19th editions thereof, which are incorporated herein by reference.

The constituents of which the combination are composed may be administered simultaneously, semi-simultaneously, separately, or spaced out over a period of time so as to obtain the maximum efficacy of the combination; it being possible for each administration to vary in its duration from a rapid administration to a continuous perfusion.

As a result, for the purposes of the present invention, the combinations are not exclusively limited to those which are obtained by physical association of the constituents, but also to those which permit a separate administration, which can be simultaneous or spaced out over a period of time.

In particular, in one embodiment of the invention, the anti-CD19 maytansinoid immunoconjugate and rituximab are administered simultaneously. In another embodiment of the invention, the anti-CD19 maytansinoid immunoconjugate and rituximab are administered sequentially. According to a particular embodiment of the invention, the anti-CD19 maytansinoid immunoconjugate is administered first, followed by administration of rituximab.

Administration of the compositions may be oral, intravenous, respiratory (e.g., nasal or intrabronchial), parenteral (besides intravenous, such as intraperitoneal and subcutaneous injections), intraperitoneal, transdermal (including all administration across the surface of the body). Preferably, the administration of the compositions according to the combination of the present invention is done intravenously. In a preferred embodiment, the anti-CD19 maytansinoid immunoconjugate is administered intravenously. In another preferred embodiment, rituximab is administered intravenously. In a further preferred embodiment, both the anti-CD19 maytansinoid immunoconjugate and rituximab are administered intravenously. However other mode of parenteral administration can be used: e.g. intramuscular, intraperitoneal or subcutaneous. While the components of the invention may be delivered via the same route, a product or pack according to the invention may contain rituximab, for delivery via a different route than that of the anti-CD19 maytansinoid immunoconjugate, e.g., one component may be delivered orally, while the other is administered intravenously. In another embodiment, rituximab and the anti-CD19 maytansinoid immunoconjugate are both delivered via the same route, e.g., intravenously. Other variations would be apparent to one skilled in the art and are contemplated within the scope of the invention.

When the combination of anti-CD19 maytansinoid immunoconjugate and rituximab is administered intravenously, it can be administered as a bolus or by continuous infusion over a period of time.

The combination of the invention may be administered in a variety of forms. These include for example liquid, semi-solid, and solid dosage forms, but the preferred form depends on the intended mode of administration and therapeutic application. Typical preferred compositions are in the form of injectable or infusible solutions. Preferred pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi.

The compositions for oral, subcutaneous or intraperitoneal administration are preferably aqueous suspensions or solutions.

The combination of the invention in the composition preferably is formulated in an effective amount. An "effective amount" refers to an amount effective, at dosages and for periods of time necessary, to achieve the desired result. A "therapeutically effective amount" means an amount sufficient to influence the therapeutic course of a particular disease state. A therapeutically effective amount is also one in which any toxic or detrimental effects of the agent are outweighed by the therapeutically beneficial effects.

Sterile compositions for parenteral administration can be prepared by incorporating the anti-CD19 maytansinoid immunoconjugate in the required amount in the appropriate solvent, followed by sterilization by microfiltration. As solvent or vehicle, there may be used water, saline, phosphate buffered saline, dextrose, glycerol, ethanol, and the like, as well as a combination thereof. In many cases, it will be preferable to include isotonic agents, such as sugars, polyalcohols, or sodium chloride in the composition. These compositions may also contain adjuvants, in particular wetting, isotonizing, emulsifying, dispersing and stabilizing agents. Sterile compositions for parenteral administration may also be prepared in the form of sterile solid compositions which may be dissolved at the time of use in sterile water or any other injectable sterile medium.

Rituximab is commercially available from GENENTECH under the names Rituxan® and MabThera® as a sterile, white to pale yellow, preservative- free lyophilized powder for intravenous (IV) administration after reconstitution with bacteriostatic saline solution for injection. Hydration with a glucocorticoid solution is required prior to each infusion with rituximab.

The examples that follow are merely exemplary of the scope of this invention and content of this disclosure. One skilled in the art can devise and construct numerous modifications to the examples listed below without departing from the scope of this invention.

**FIGURE LEGENDS:**

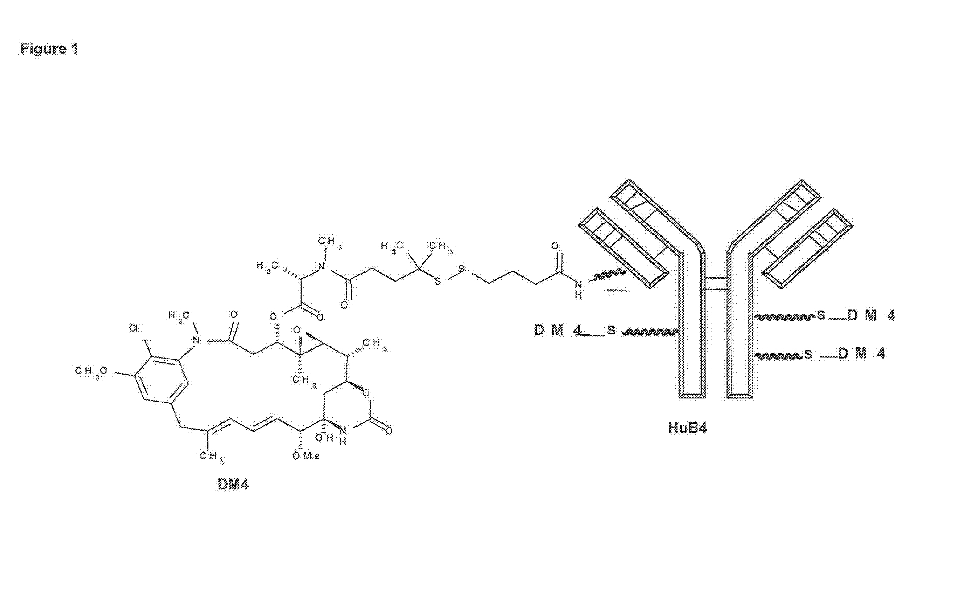

**Figure 1:** Structure of the anti-CD19 maytansinoid immunoconjugate.

**Figure 2:** Representative sequential FDG-PET images (coronal view) of mice bearing disseminated WSU-DLCL2 human lymphoma, treated with SAR3419, rituximab or a combination of both agents. The white arrows point the inoculated tumors.

**Figure 3:** Tumor metabolic responses to SAR3419, rituximab and SAR3419/rituximab combination as a function of time, in mice bearing disseminated WSU-DLCL2 human lymphoma.

**Figure 4:** Survival curves of WSU-DLCL2-inoculated mice treated with SAR3419, rituximab or a combination of both agents.

**EXAMPLES**

**MicroPET imaging and survival studies on the disseminated model of human lymphoma WSU-DLCL2, combination studies with rituximab**

**Introduction**

Therapeutic activity of a combination of SAR3419 and rituximab was assessed in the disseminated model of human lymphoma WSU-DLCL2. The WSU-DLCL2 cell line is considered a chemotherapy-resistant model since it was established from a patient with aggressive lymphoma refractory to therapy (Al-Katib et al., Clin Cancer Res., 4(5): 1305-1314, 1998; Al-Katib et al., Clin Cancer Res., 15(12):4038-4045, 2009).

Metabolic imaging with positron emission tomography (PET) using fluorine-18 labelled fluorodeoxyglucose (FDG) as the tracer (hereafter FDG-PET), is widely accepted for staging of lymphoma patients and assessment of response after completion of therapy. In addition it is now being used for monitoring response during treatment in lymphoma patients (Juweid, Methods Mol Biol., 727: 1-19, 2011). FDG-PET was used for longitudinal non-invasive monitoring of tumor metabolism in SCID mice bearing the WSU-DLCL2 human lymphoma, treated with SAR3419 combined to rituximab. Survival rate was also studied by evaluating the increase in life span (ILS) of SAR3419/rituximab.

**Experimental procedure**

Animals or biological material

*Animals*

CB17/lcr-Prkdcscid mice/crl (SCID), at 8-10 weeks old, were bred at Charles River France (Domaine des Oncins, 69210 L'Arbresle, France) from strains obtained from Charles River, USA.

Mice were over 17 g at start of chemotherapy after an acclimatization time of at least 5 days. They had free access to food (UAR reference 113, Villemoisson, 91160 Epinay sur Orge, France) and water. They were housed on a 12 h light/dark cycle. Environmental conditions including animal maintenance, room temperature ($22°C \pm 2°C$), relative humidity ($55\% \pm 15\%$) and lighting times were recorded by the supervisor of animal research and welfare (ARW).

*Tumor model*

Diffuse Large Cell Lymphoma Cell Line WSU-DLCL2 was established at Wayne State University from a patient with relapsed and resistant diffuse large cell lymphoma. The WSU-DLCL2 cell line was shown to express both CD19 and CD20 target antigens (Al-Katib et al., Clin Cancer Res., 15(12):4038-4045, 2009).

Procedure

*Preparation for intravenous treatment*

For intravenous (IV) injections, compounds were dissolved in Glucose 5% in water. Drugs were kept at room temperature and administered as a bolus within few minutes after formulation. The volume of injection was 10 mL/kg.

*Study design: chemotherapy and PET examination*

MicroPET scans were done using the microPET FOCUS 120 PET scanner (Siemens Preclinical Solutions, Knoxville, TN). Mice were fasted for 14 to 16 hours prior to FDG injection and placed on a heating pad (30-33°C) starting 30 minutes prior to tracer injection. Mice were not anesthetized for FDG injection and kept conscious during FDG uptake. Imaging was performed on isoflurane-anesthetized mice starting 60 minutes after an IV injection of 5 to 7 MegaBecquerel (MBq) of FDG.

The pattern of tumor invasion after IV inoculation of WSU-DLCL2 human lymphoma cells in SCID mice had been previously characterized using FDG-PET. At an advanced stage of disease, involvement of kidney, ovary and spinal cord sites could be evidenced in a majority of mice enabling the use of the baseline PET signal for distribution of mice into balanced treatment groups.

*SAR3419 in combination with rituximab randomized on PET signal with imaging and survival endpoints*

On day 0, the animals were injected with 3 millions of WSU-DLCL2 cells. The tumors were allowed to grow to the desired size range upon FDG uptake signal measured with the microPET; Glycolytic tumor metabolism was assessed prior to the drug injection (baseline on days 47 or 48), animals with non-detectable tumors were excluded. The mice were then pooled and randomized into 3 treatment groups (n = 6) and 1 vehicle group (n = 6). The mice in the treatment groups were administered on days 49 and 57 with SAR3419 (20 mg/kg), rituximab (20 mg/kg) or the combination of both agents. The mice in the control group were injected with vehicle on the same days. Injections were performed as a single bolus injection under 10 mL/kg via the IV route. The FDG-PET sessions were then performed serially from day 55 to day 96 (6 imaging sessions overall post-treatment).

Survival rate was examined everyday up to 137 days post tumor inoculation.

*End points for assessing host toxicity*

Drug-related host toxicity is defined as at least 20% body weight loss (BWL). Paralysis of the hind legs is also considered as a sign of over toxicity.

*End points for assessing antitumor activity*

*Assessment of tumor metabolism from PET images*

Image acquisition time was 12 minutes. Images were reconstructed using 2-D ordered subset-expectation maximization reconstruction algorithm (OSEM2D). Regions of interest were manually drawn over the tumor sites using the image analysis software ASIPro (Concorde Microsystems). The volume of interest (in cm$^3$) (VOI) was generated by stacking two-dimensional regions of interest (in cm$^2$) within consecutive planes. Tracer uptake in the VOI was expressed as standardized uptake value (SUV) using the formula:

$$SUV = \frac{Tumor\ activity\ concentration\ (MBq/ml)}{Injected\ dose\ (MBq)} \times body\ weight\ (g)$$

When multiple sites were invaded by the tumor, the reported SUV was the sum of SUVs measured in the different VOIs.

*Assessment of survival rate*

In the assessment of tumor-related mortality, mice were scored as dead when they were either found dead or sacrificed when one of the following criteria was observed: complete loss of mobility due to paralysis of the posterior legs, excessive loss of weight (>20%) or 10% drug related deaths, tumor mass >2g or any tumor development preventing animal mobility. Drug efficacy was determined by evaluating the ILS after tumor implantation. ILS is expressed as % ratio of median day of death in treated-animal groups (T) compared to the median day of death of control animals (C) and is calculated using the following formula:

$$\%ILS = \frac{(T-C)}{C} \times 100$$

<u>Statistical analysis</u>

SAS V8.2 for Sun Solaris via Everstat V5.0 interface and SAS V9.1 were used to perform the statistical analyses on individual data.

For SUV parameter, $\Delta SUV = \dfrac{SUV_t}{SUV_{t_{48}}} \times 100$ was calculated for each animal at each time (t), and descriptive statistics (mean $\pm$ sd) were given for each group at each day.

Survival curves were plotted according to the Kaplan-Meier method, and survival distribution across treatment groups was compared using log-rank test analyses.

The significance level of statistical tests was chosen to be 5% (p-value <0.05).

**Results**

<u>SAR3419 in combination with rituximab randomized on PET signal with imaging and survival endpoints</u>

In this experiment, mice bearing disseminated WSU-DLCL2 at an advanced stage of disease were treated on a q8dx2 schedule (day 49 and day 57) with 20 mg/kg SAR3419 (total dose 40 mg/kg), 20 mg/kg rituximab (total dose 40 mg/kg) or a combination of both agents (same dosages). The groups were constituted on the basis of the PET signal at baseline. The temporal changes in tumor metabolism upon treatment were followed using FDG-PET and the impact of treatment regimen on mice survival was assessed. All treatments were well tolerated with no overt toxicity and a maximum BWL of 9% for SAR3419 single agent.

*Temporal changes of FDG uptake upon treatment*

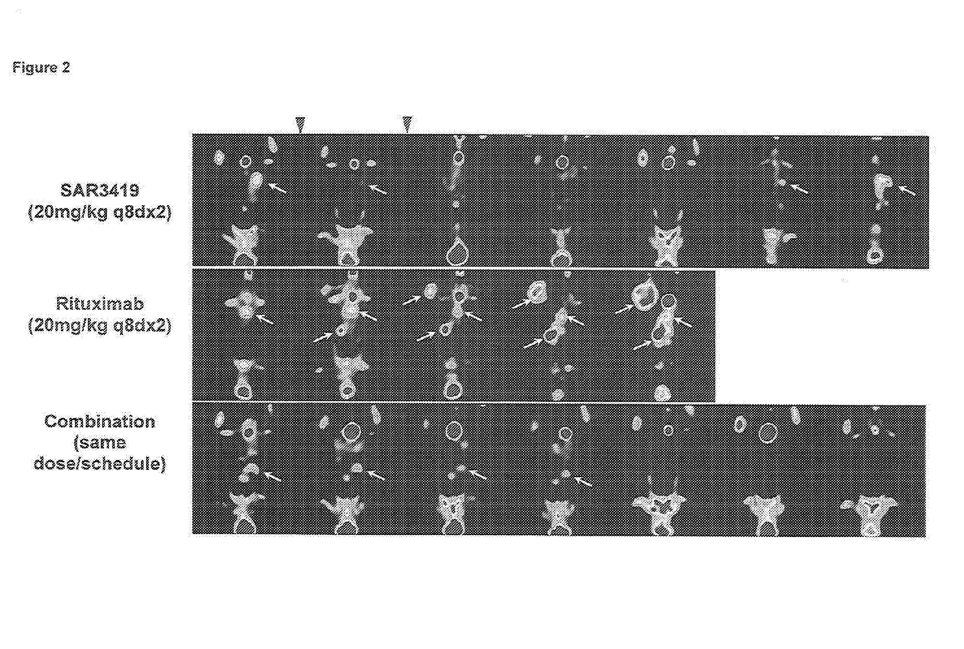

A representative set of FDG-PET images taken from one animal of each treated group is shown in Figure 2. Qualitatively, a decrease in FDG uptake along time is observed for the SAR3419 group (with maximum regression on day 69 and recurrence on day 83), but not for the rituximab group. In the combination group, the decrease in FDG uptake is delayed with respect to SAR3419 single agent, but persists over time (no observable PET signal on day 96).

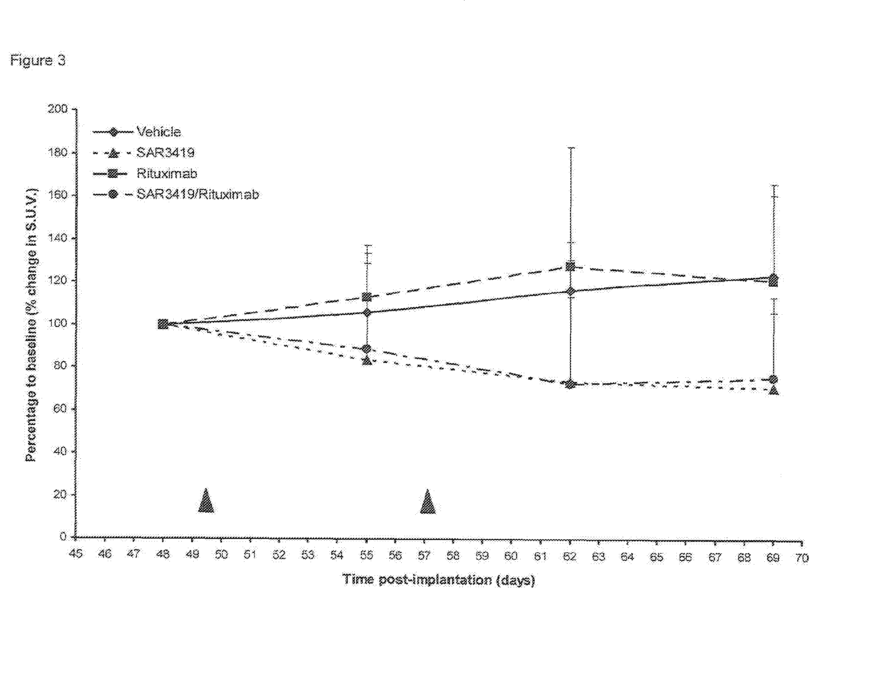

The quantitative analysis of SUV measured for the control and treatment groups from day 48 to day 69 after tumor implantation, is presented in Table 1 and variations from baseline values are illustrated in Figure 3. The FDG uptake is decreased in SAR3419-treated animals (alone or in combination with rituximab) when compared to vehicle-treated animals. Following the first administration on day 49, a 33% decrease of tumor metabolism in SAR3419-treated animals and a 26 % decrease in SAR3419/rituximab-treated animals are observed 6 days post-treatment compared to vehicle-treated animals. Following the second administration on day 57, the metabolic activity is reduced by 47 % in the SAR3419 group and by 42 % in the combination group compared to vehicle group. On day 69 (i.e. 12 days post-second treatment), the metabolic activity is reduced by 41% in the SAR3419 group and by 36 % in the combination group compared to vehicle group.

**Table 1:** Evaluation of SAR3419 in combination with rituximab against disseminated WSU-DLCL2 human lymphoma in female SCID mice: results

| Group (Size) | Agent (batch) | Route/ Dosage in mL/kg per injection | Dosage in mg/kg per injection (total dose) | Schedule in days | Drug death (Day of death) | Average BWL in % per mouse at nadir (day of nadir) | Imaging schedule in days | SUV Day: mean (± SD) | ΔSUV relative to day 48 baseline in % Day: mean (± ΣΔ) | Increase Life Span in % | Statistical analysis p value |
|---|---|---|---|---|---|---|---|---|---|---|---|
| 1(6) | Vehicle | IV 10mL/kg | - | 49, 57 | 0/6 | 7 3% (61) | 48, 55, 62, 69 | 48 2 59 (0 64)<br>55 2 56 (0 59)<br>62 2 76 (1 06)<br>69 2 45 (0 50) | 48 100<br>55 106 (23)<br>62 116 (27)<br>69 124 (43) | - | - |
| 2(6) | SAR3419 (DI01250) | IV 10mL/kg | 20 (40) | 49, 57 | 0/6 | 8 9% (62) | 48, 55, 62, 69 | 48 2 22 (0 54)<br>55 1 72 (0 21)<br>62 1 47 (0 76)<br>69 1 46 (0 78) | 48 100<br>55 83 (29)<br>62 73 (40)<br>69 71 (35) | 41% | NS |
| 3(6) | rituximab (702355) | IV 10mL/kg 20 (40) 49, 57 0/6 1 6% (62) 48, 55, 62, 69 | 20 (40) | 49, 57 | 0/6 | 1 6% (62) | 48, 55, 62, 69 | 48 3 08 (1 08)<br>55 3 37 (0 97)<br>62 3 59 (1 35)<br>69 3 47 (1 17) | 48 100<br>55 113 (25)<br>62 128 (55)<br>69 121 (40) | 13% | NS |

| Group (Size) | Agent (batch) | Route/ Dosage in mL/kg per injection | Dosage in mg/kg per injection (total dose) | Schedule in days | Drug death (Day of death) | Average BWL in % per mouse at nadir (day of nadir) | Imaging schedule in days | SUV Day: mean (± SD) | ΔSUV relative to day 48 baseline in % Day: mean (± ΣΔ) | Increase Life Span in % | Statistical analysis p value |
|---|---|---|---|---|---|---|---|---|---|---|---|
| 4(6) | SAR3419/rituximab | IV 10mL/kg | 20/20 (40/40) | 49, 57 | 0/6 | 81% (62) | 48, 55, 62, 69 | 48 2 05 (0 31) 55 1 88 (0 98) 62 1 60 (1 27) 69 1 57 (0 78) | 48 100 55 88 (45) 62 72 (58) 69 75 (38) | 83% | NS |

Mice average weight Due to body weight heterogeneity (range Vehicle =18 62 - 23 75 g, SAR3419 =17 15 - 23 61 g, rituximab = 20 11 - 24 08 g, SAR3419/rituximab= 19 03 - 21 91 g) dosages were adjusted to the individual body weights Drug formulation SAR3419= Glucose 5% in water, rituximab= Glucose 5% in water Treatment duration 2 injections overall Imaging protocol first image acquisition was performed before any treatment Mice were thermo-regulated throughout the study Fasting period 16-20h prior to FDG tracer injection Mean activity injected per animal = 6 61 ± 0 42 MBq (Mean ± SD) Image acquisition time was 12 min, 60 min post tracer injection Imaging performed on isoflurane-anesthetized mice Images were reconstructed using OSEM2D algorithm The tumoral FDG uptake is expressed as SUV Statistical analysis survival distribution across treatment groups was compared using log-rank test analyses A probability less than 5% (p<0 05) was considered as significant No significance was observed between the four groups, p=0 2462 Abbreviations used BWL = body weight loss, SUV = standardized uptake value, SD = standard deviation, FDG = ([$^{18}$F]-fluorodeoxyglucose, MBq = megabecquerel, OSEM2D = 2-D Ordered Subset-Expectation Maximization reconstruction algorithm, ILS = increase in life span

*Effect of treatment on survival rate*

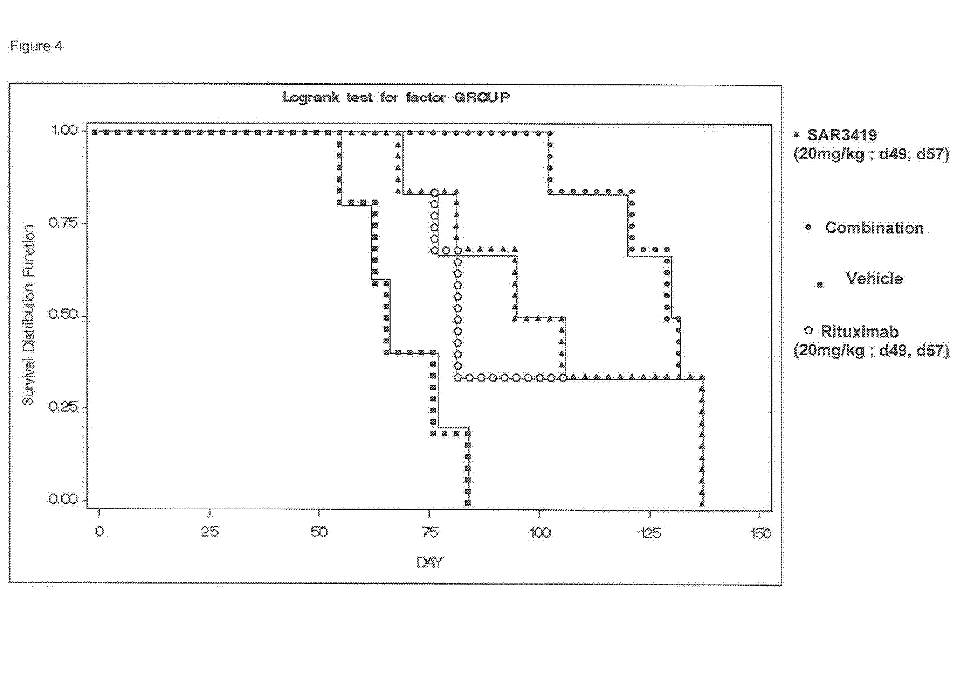

The survival data are presented in Table 1 and illustrated in Figure 4. The combination of SAR3419 and rituximab improves the survival with 83% ILS. The survival is also improved in single agent groups but to a lesser extend: 41% ILS for SAR3419 and 13% ILS for rituximab. Significance was not reached for any of these comparisons.

**Conclusion**

The antitumor efficacy of the drug combination of SAR3419 with rituximab was evaluated in a disseminated model of human CD19+/CD20+ DLBCL. Immunodeficient mice were inoculated with WSU-DLCL2 cells and disease progression was monitored using serial FDG-PET. The mice were treated at an advanced stage of disease with SAR3419, rituximab or the combination of both agents (20 mg/kg in a q8dx2 schedule by the IV route). The endpoint for efficacy was the increase of life span (ILS) in treated groups with respect to the control group. When SAR3419 was combined with rituximab, the decrease in FDG tumor uptake was sustained over time and translated into an 83% ILS to be compared with 41% and 13% ILS for groups receiving SAR3419 and rituximab single agents, respectively. Overall, combining the anti-CD19 SAR3419 with the anti-CD20 rituximab improved mice survival in a disseminated model of human DLBCL expressing both molecular targets.

**Example 2: Study of intravenous SAR3419 in combination with rituximab in patients with relapsed or refractory Diffuse Large B Cell lymphomas**

**Study objective(s)**

Primary Objective:

• To evaluate the overall response rate of SAR3419 in combination with rituximab

Secondary Objective(s):

• To assess the safety profile of the combination

• To evaluate the efficacy of the combination in terms of duration of the response (RD), progression free survival (PFS) and overall survival (OS)

• To determine the pharmacokinetics (PK) of SAR3419, of its metabolites DM4 and Me-DM4 (non protein-bound maytansinoids) and of rituximab, when administered in combination.

• To determine the immunogenicity of SAR3419

Exploratory objective:

To characterize patients' tumor tissue and correlate antitumor and biological activity of the combination with biomarker status.

**Methods**

Study design

This is an open label, multicenter phase II study of SAR3419 administered in combination with rituximab in patients with relapsed or refractory Diffuse Large B-cell lymphomas (DLBCL).

Adult patients with refractory or relapsed CD19+ CD20+ DLBCL are enrolled. In particular, inclusion criteria include after refractory or relapsed at least one standard treatment including rituximab for aggressive lymphoma and not candidate or not eligible for high dose chemotherapy with stem cell support (refractory disease is defined as unresponsive to a standard regimen or progressing within six months of completing a standard regimen) or relapsed after high dose chemotherapy with autologous stem cell support.

SAR3419 and rituximab is administered intravenously at 55mg/m$^2$ and 375mg/m$^2$ respectively, weekly, on weeks 1 to 4, then bi-weekly, on weeks 6, 8, 10, 12. One cycle is defined as a 4-week period except for cycle 1 which should last 5 weeks (4 weekly administrations followed by one week rest).

The combined therapy is provided for 8 doses in the absence of unacceptable toxicity, disease progression or withdrawal of consent.

Evaluations

*Efficacy data*

Disease assessment using CT / PET scan is performed at baseline, after the last study treatment dose and at the end of treatment (EOT, 42 days after the last administration of study treatment). PET is repeated at the end of treatment only to confirm a CR. If not performed within the prior 3 months before inclusion, bone marrow biopsy is done at baseline. It is repeated only if involved at baseline for confirming a complete response (CR). At the end of therapy, responders continue to have tumor assessments every 12 weeks until documented progressive disease or initiation of a new lymphoma-specific therapy, whichever occurs earlier. All patients are followed for survival for at least 24 months.

*Safety data*

Vital signs, physical examination, ophthalmologic assessment, ECOG Performance Status (PS), laboratory safety tests, are obtained prior to drug administration and at designated intervals throughout the study in all patients. Pharmacokinetics are performed at designated intervals during the treatment.

Tumor tissue samples are used for characterizing the patients' tumor and making correlation with disease outcome.

**Results**

The combination of SAR3419 and rituximab shows high efficacy against refractory or relapsed CD19+ CD20+ DLBCL. In addition, the composition of the invention shows reduced ocular toxicity.

**Claims**

1.  A combination of rituximab and an anti-CD19 maytansinoid immunoconjugate for use in treating B-cell malignancies symptom in a patient in need thereof.

2.  The combination of claim 1, wherein the said B-cell malignancies symptom is a CD19+ CD20+ B-cell malignancies symptom.

3.  The combination of any one of claims 1 or 2, wherein said CD19+ CD20+ B-cell malignancies symptom is a leukemia symptom or a lymphoma symptom.

4.  The combination of any of claims 1 to 3, wherein said leukemia symptom is Non-Hodgkin's lymphoma symptom (NHL) symptom.

5.  The combination of any of claims 1 to 3, wherein said lymphoma symptom is Acute lymphoblastic leukemia (ALL) symptom.

6.  The combination of claim 4, wherein said Non-Hodgkin's lymphoma symptom is a Diffuse Large B-cell lymphoma (DLBCL), a follicular lymphoma (FL), a Mantle cell lymphoma (MCL), a Marginal zone lymphoma (MZL), or a Small lymphocytic lymphoma (SLL).

7.  The combination of claim 4, wherein said Non-Hodgkin's lymphoma symptom is a relapsed or refractory B-cell non-Hodgkin's lymphoma.

8.  The combination of claim 4, wherein said Non-Hodgkin's lymphoma symptom is a B-cell non-Hodgkin's lymphoma expressing CD19 and CD20.

9.  The combination of claim 4, wherein said patient has already been treated for the Non-Hodgkin's lymphoma symptom.

10. The combination of claim 4, wherein said patient has failed rituximab therapy.

11. The combination of claim 4, wherein said Non-Hodgkin's lymphoma symptom is a rituximab resistant disease.

12. The combination of claim 4, wherein said patient has received an autologous or allogeneic stem cell transplant.

13. The combination of any of claims 1 to 12, wherein the said anti-CD19 maytansinoid immunoconjugate comprises an antibody which binds specifically to the CD19 antigen conjugated to a cytotoxic of the maytansinoid family.

14. The combination of any of claims 1 to 13, wherein the anti-CD19 maytansinoid immunoconjugate comprises an antibody which binds specifically to the CD19 antigen conjugated to DM4.

15. The combination of any of claims 1 to 14, wherein the anti-CD19 maytansinoid immunoconjugate comprises an antibody which binds specifically to the CD19 antigen conjugated to DM4 through a cleavable linker.

16. The combination of any of claims 1 to 15, wherein the anti-CD19 maytansinoid immunoconjugate comprises an antibody which binds specifically to the CD19 antigen conjugated to DM4 through SPDB.

17. The combination of any of claims 1 to 16, wherein the said antibody comprises six complementary determining region (CDR), said CDR having the sequences represented in SEQ ID NOs 1 to 6.

18. The combination of any of claims 1 to 17, wherein the said antibody comprises a light chain, wherein the sequence of the said light chain has at least 60%, preferably of at least 75%, more preferably at least 85%, still more preferably 95 % and even more preferably of at least 99% identity with the sequence displayed in SEQ ID NO. 7.

19. The combination of any of claims 1 to 18, wherein the said antibody comprises a heavy chain, wherein the sequence of the said heavy chain has at least 60%, preferably of at least 75%, more preferably at least 85%, still more preferably 95 % and even more preferably of at least 99% identity with the sequence displayed in SEQ ID NO. 8.

20. The combination of any of claims 1 to 19, wherein the said antibody comprises a light chain and a heavy chain, said light chain having the sequence represented in SEQ ID NO. 7, and said heavy chain having the sequence represented in SEQ ID NO. 8.

21. The combination of any of claims 1 to 20, wherein the anti-CD19 maytansinoid immunoconjugate comprises an antibody which binds specifically to the CD19 antigen conjugated to DM4 through SPDB wherein almost 3.5 molecules of DM4 are bound through the SPDB linker to each huB4 molecule.

22. The combination of any of the claims 1 to 21, wherein the said anti-CD19 maytansinoid immunoconjugate and rituximab are administered simultaneously, separately or sequentially.

23. The combination of any of the claims 1 to 22, wherein the said anti-CD19 maytansinoid immunoconjugate is administered intravenously.

24. The combination of any of the claims 1 to 23, wherein rituximab is administered intravenously.

25. The combination of any of the claims 1 to 24, wherein the said anti-CD19 maytansinoid immunoconjugate and rituximab are administered intravenously.

26. The combination of any of the claims 1 to 25, wherein:

(a) an initial dose of almost 55 mg/m$^2$ of the anti-CD19 maytansinoid immunoconjugate and of almost 375 mg/m$^2$ of rituximab, is first administered to the patient; and

(b) a plurality of subsequent doses of almost 55 mg/m$^2$ of the anti-CD19 maytansinoid immunoconjugate of almost 375 mg/m$^2$ of rituximab, are then administered to the patient, each administration being separated in time from the other by one week.

27. The combination of claim 26, comprising a further step (c) of administering a plurality of subsequent doses of almost 55 mg/m$^2$ of the anti-CD19 maytansinoid immunoconjugate of almost 375 mg/m$^2$ of rituximab, each administration being separated in time from the other by two weeks.

28. The combination of any of the claims 1 to 27, wherein:

(a) an initial dose of almost 55 mg/m$^2$ of the anti-CD19 maytansinoid immunoconjugate and of almost 375 mg/m$^2$ of rituximab, is first administered to the patient;

(b) 3 subsequent doses of almost 55 mg/m$^2$ of the anti-CD19 maytansinoid immunoconjugate of almost 375 mg/m$^2$ of rituximab, are then administered to the patient, each administration being separated in time from the other by one week; and

(c) 4 subsequent doses of almost 55 mg/m$^2$ of the anti-CD19 maytansinoid immunoconjugate of almost 375 mg/m$^2$ of rituximab, are further administered to the patient, each administration being separated in time from the other by two weeks.

29. A pharmaceutical pack containing a course of a treatment against CD19+ CD20+ malignancies symptom for one individual patient, wherein the pack contains (a) at least one unit of rituximab and (b) at least one unit of the anti-CD19 maytansinoid immunoconjugate in unit dosage form.

Figure 1

DM4

HuB4

EP 2 550 975 A1

Figure 2

SAR3419
(20mg/kg q8dx2)

Rituximab
(20mg/kg q8dx2)

Combination
(same
dose/schedule)

Figure 3

22

Figure 4

Logrank test for factor GROUP

▲ SAR3419
(20mg/kg ; d49, d57)

⊕ Combination

▣ Vehicle

○ Rituximab
(20mg/kg ; d49, d57)

Europäisches
Patentamt
European
Patent Office
Office européen
des brevets

**EUROPEAN SEARCH REPORT**

Application Number

EP 11 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TASSONE PIERFRANCESCO ET AL: "Cytotoxic activity of the maytansinoid immunoconjugate B-B4-DM1 against CD138+ multiple myeloma cells", BLOOD, AMERICAN SOCIETY OF HEMATOLOGY, US, vol. 104, no. 12, 1 December 2004 (2004-12-01), pages 3688-3696, XP002521112, ISSN: 0006-4971, DOI: 10.1182/BLOOD-2004-03-0963 [retrieved on 2004-08-03] * abstract * * page 3689, left-hand column, paragraph 2 * * page 3690, left-hand column, paragraph 1 - paragraph 2 * * page 3691, left-hand column, last paragraph - page 3694 * * page 3695, left-hand column, last paragraph - right-hand column, line 2 * ----- | 1-29 | INV. A61K47/48 A61K39/395 C07K16/28 ADD. A61P35/00 |
| Y | DATABASE BIOSIS [Online] BIOSCIENCES INFORMATION SERVICE, PHILADELPHIA, PA, US; November 2009 (2009-11), YOUNES ANAS ET AL: "Phase I Multi-Dose Escalation Study of the Anti-CD19 Maytansinoid Immunoconjugate SAR3419 Administered by Intravenous (IV) Infusion Every 3 Weeks to Patients with Relapsed Refractory B-Cell Non-Hodgkin's Lymphoma (NHL)", XP002659847, Database accession no. PREV201000351579 * abstract * -/-- | 1-29 | TECHNICAL FIELDS SEARCHED (IPC) A61K C07K |

The present search report has been drawn up for all claims

| Place of search | Date of completion of the search | Examiner |
|---|---|---|
| The Hague | 21 November 2011 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 11 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & BLOOD, vol. 114, no. 22, November 2009 (2009-11), page 243, 51ST ANNUAL MEETING OF THE AMERICAN-SOCIETY-OF-HEMATOLOGY; NEW ORLEANS, LA, USA; DECEMBER 05 -08, 2009 ISSN: 0006-4971(print) ----- | | |
| Y | US 5 208 020 A (CHARI RAVI J [US] ET AL) 4 May 1993 (1993-05-04) * examples 3,5 * ----- | 1-29 | |
| Y | WO 2004/103272 A2 (IMMUNOGEN INC [US]; WIDDISON WAYNE C [US]; CHARI RAVI V J [US]) 2 December 2004 (2004-12-02) * examples 12-14 * ----- | 1-29 | |
| Y | LOCK R ET AL: "Pediatric Preclinical Testing Program (PPTP) evaluation of the anti-CD19-DM4 conjugated antibody SAR3419", EUROPEAN JOURNAL OF CANCER, SUPPLEMENT, vol. 6, no. 12, 192, 1 October 2008 (2008-10-01), page 61, XP025534254, ISSN: 1359-6349, DOI: 10.1016/S1359-6349(08)72124-4 [retrieved on 2008-10-01] * abstract * ----- -/-- | 1-29 | TECHNICAL FIELDS SEARCHED (IPC) |

The present search report has been drawn up for all claims

| Place of search | Date of completion of the search | Examiner |
|---|---|---|
| The Hague | 21 November 2011 | Dullaart, Anwyn |

Europäisches
Patentamt

European
Patent Office

Office européen
des brevets

# EUROPEAN SEARCH REPORT

**Application Number**

EP 11 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Bertrand Coiffier et al.: "Phase I/II study of the anti-CD19 maytansinoid immunoconjugate SAR3419 administered weekly to patients (pts) with relapsed/refractory B-cell non-Hodgkin lymphoma (NHL).", ASCO MEETING ABSTRACTS 2011 JOURNAL OF CLINICAL ONCOLOGY, vol. 29, no. 15, suppl., 8017, 9 June 2011 (2011-06-09), XP002663986, Retrieved from the Internet: URL:http://www.asco.org/ASCOv2/Meetings/Ab stracts?&vmview=abst_detail_view&confID=10 2&abstractID=78324 [retrieved on 2011-11-16] * abstract * | 1-29 | |
| Y | DAVID J. FLAVELL ET AL: "The anti-CD20 antibody rituximab augments the immunospecific therapeutic effectiveness of an anti-CD19 immunotoxin directed against human B-cell lymphoma", BRITISH JOURNAL OF HAEMATOLOGY, vol. 134, no. 2, 1 July 2006 (2006-07-01), pages 157-170, XP55012172, ISSN: 0007-1048, DOI: 10.1111/j.1365-2141.2006.06155.x * abstract * * page 161 - page 162 * * figure 4 * | 1-29 | TECHNICAL FIELDS SEARCHED (IPC) |

-/--

The present search report has been drawn up for all claims

| Place of search | Date of completion of the search | Examiner |
|---|---|---|
| The Hague | 21 November 2011 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 11 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ABOUKAMEEL A ET AL: "Superior anti-tumor activity of the CD19-directed immunotoxin SAR3419 to rituximab in non-Hodgkin's xenograft animal models: preclinical evaluation", BLOOD, AMERICAN SOCIETY OF HEMATOLOGY, US, vol. 110, 1 January 2007 (2007-01-01), page 1, XP008145317, ISSN: 0006-4971 * abstract * | 1-29 | |
| Y | GERBER HANS-PETER ET AL: "Potent antitumor activity of the anti-CD19 auristatin antibody drug conjugate hBU12-vcMMAE against rituximab-sensitive and -resistant lymphomas", BLOOD, AMERICAN SOCIETY OF HEMATOLOGY, US, vol. 113, no. 18, 1 April 2009 (2009-04-01), pages 4352-4361, XP009151913, ISSN: 0006-4971, DOI: 10.1182/BLOOD-2008-09-179143 [retrieved on 2009-01-15] * abstract; figures * * page 4354, right-hand column * * page 4359, left-hand column - page 4360, right-hand column * | 1-29 | TECHNICAL FIELDS SEARCHED (IPC) |

-/--

The present search report has been drawn up for all claims

| Place of search | Date of completion of the search | Examiner |
|---|---|---|
| The Hague | 21 November 2011 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 11 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | A. M. AL-KATIB ET AL: "Superior Antitumor Activity of SAR3419 to Rituximab in Xenograft Models for Non-Hodgkin's Lymphoma", CLINICAL CANCER RESEARCH, vol. 15, no. 12, 9 June 2009 (2009-06-09), pages 4038-4045, XP55012064, ISSN: 1078-0432, DOI: 10.1158/1078-0432.CCR-08-2808 * abstract * * page 4038, right-hand column * ----- | 1-29 | |
| Y | FLAVELL DAVID J ET AL: "Delineation of the cytotoxic mechanisms operating in SCID mice xenografted with human B-cell lymphoma following treatment with a combination of anti-CD20 antibody plus anti-CD19 immunotoxin", PROCEEDINGS OF THE AMERICAN ASSOCIATION FOR CANCER RESEARCH ANNUAL MEETING, vol. 42, 1538, March 2001 (2001-03), page 286, XP002663987, & 92ND ANNUAL MEETING OF THE AMERICAN ASSOCIATION FOR CANCER RESEARCH; NEW ORLEANS, LA, USA; MARCH 24-28, 2001 ISSN: 0197-016X * abstract * ----- -/-- | 1-29 | TECHNICAL FIELDS SEARCHED (IPC) |

The present search report has been drawn up for all claims

| Place of search | Date of completion of the search | Examiner |
|---|---|---|
| The Hague | 21 November 2011 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                
& : member of the same patent family, corresponding document

EPO FORM 1503 03.82 (P04C01)

# EP 2 550 975 A1

**EUROPEAN SEARCH REPORT**

Application Number

EP 11 30 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FLAVELL DAVID J ET AL: "Co-operative therapeutic effects between anti-CD20 antibodies and an anti-CD19 immunotoxin in a SCID mouse model of human B-cell lymphoma", PROCEEDINGS OF THE AMERICAN ASSOCIATION FOR CANCER RESEARCH ANNUAL MEETING, no. 41, 523, March 2000 (2000-03), page 82, XP002663988, & 91ST ANNUAL MEETING OF THE AMERICAN ASSOCIATION FOR CANCER RESEARCH.; SAN FRANCISCO, CALIFORNIA, USA; APRIL 01-05, 2000 ISSN: 0197-016X * abstract * ----- | 1-29 | |

TECHNICAL FIELDS
SEARCHED (IPC)

The present search report has been drawn up for all claims

| Place of search | Date of completion of the search | Examiner |
|---|---|---|
| The Hague | 21 November 2011 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

29

## ANNEX TO THE EUROPEAN SEARCH REPORT
## ON EUROPEAN PATENT APPLICATION NO.

EP 11 30 5999

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2011

| Patent document cited in search report | | Publication date | Patent family member(s) | | Publication date |
|---|---|---|---|---|---|
| US 5208020 | A | 04-05-1993 | US 5208020 A | | 04-05-1993 |
| | | | US 5416064 A | | 16-05-1995 |
| WO 2004103272 | A2 | 02-12-2004 | AU 2004240541 A1 | | 02-12-2004 |
| | | | BR PI0410748 A | | 27-06-2006 |
| | | | CA 2525130 A1 | | 02-12-2004 |
| | | | CN 1956722 A | | 02-05-2007 |
| | | | CN 101186613 A | | 28-05-2008 |
| | | | CO 5660276 A2 | | 31-07-2006 |
| | | | EC SP056149 A | | 17-10-2006 |
| | | | EP 1651162 A2 | | 03-05-2006 |
| | | | JP 2007514646 A | | 07-06-2007 |
| | | | KR 20060003120 A | | 09-01-2006 |
| | | | MX PA05011811 A | | 17-02-2006 |
| | | | NZ 542695 A | | 30-04-2009 |
| | | | WO 2004103272 A2 | | 02-12-2004 |
| | | | ZA 200507845 A | | 28-03-2007 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5736137 A

- US 20040235840 A


**Non-patent literature cited in the description**

- **MCLAUGHLIN et al.** *J Clin Oncol,* 1998, vol. 16, 2825-2833
- **DAVIS et al.** *J Clin Oncol,* 2000, vol. 18, 3135-3143
- **ROGUSKA et al.** *Proc. Natl. Acad. Sci. USA,* 1994, vol. 91, 969-973
- Remington's Pharmaceutical Science. Mack Publishing Company, 1985

- **AL-KATIB et al.** *Clin Cancer Res.,* 1998, vol. 4 (5), 1305-1314
- **AL-KATIB et al.** *Clin Cancer Res.,* 2009, vol. 15 (12), 4038-4045
- **JUWEID.** *Methods Mol Biol.,* 2011, vol. 727, 1-19